# EUROPÄISCHE PATENTSCHRIFT

(11) **EP 3 579 904 B1**
(45) Veröffentlichungstag und Bekanntmachung des Hinweises auf die Patenterteilung: **10.11.2021**
(21) Anmeldenummer: 18704952.3
(22) Anmeldetag: 08.02.2018
(51) Int. Cl.: A61M 25/00, A61M 60/135, A61M 60/205, A61M 60/829, A61M 25/04

(54) **KATHETERPUMPE MIT ANTRIEBSEINHEIT UND KATHETER**
CATHETER PUMP COMPRISING DRIVE UNIT AND CATHETER
POMPE À CATHÉTER DOTÉE D'UNE UNITÉ D'ENTRAÎNEMENT ET D'UN CATHÉTER

(30) Priorität: 13.02.2017 DE 102017102824
(43) Veröffentlichungstag der Anmeldung: 18.12.2019
(73) Patentinhaber: Cardiobridge GmbH, 72379 Hechingen (DE)
(72) Erfinder: EPPLE, Klaus, 72414 Rangendingen (DE)
(74) Vertreter: DREISS Patentanwälte PartG mbB
(86) Internationale Anmeldenummer: PCT/EP2018/053131
(87) Internationale Veröffentlichungsnummer: WO 2018/146177

(56) Entgegenhaltungen:
- EP-A1- 2 246 078
- EP-A1- 2 745 869
- WO-A1-2015/187838
- DE-A1-102013 011 042
- US-A1- 2011 282 128

## Beschreibung

Die Erfindung betrifft eine Katheterpumpe mit einem Katheter, mit einem am distalen Ende des Katheters vorgesehenen Pumpenkopf zum Einführen in das arterielle Gefäßsystem, insbesondere in die Aorta oder das Herz, wobei der Katheter einen Außenkatheter und einen im Außenkatheter angeordneten Innenkatheter aufweist, mit einer im Innenkatheter verdrehbar angeordneten Rotorwelle zum Antreiben eines am Pumpenkopf vorgesehenen, expandierbaren Förderelements, mit einem am proximalen Ende des Katheters vorgesehenen Betätigungsabschnitt, über den die Rotorwelle antreibbar ist, mit einem das Förderelement umgebenden Käfig, wobei der Käfig eine distale und eine proximale Hülse sowie zwischen den Hülsen verlaufende Filamente aufweist, wobei die proximale Hülse zum Expandieren des Käfigs in axialer Richtung hin zur distalen Hülse bewegt werden kann. Dabei expandieren die zwischen den Hülsen liegenden Bereiche der Filamente nach radial außen, um einen das expandierende Förderelement umgebenden Raum zu bilden.

Derartige Katheterpumpen sind beispielsweise aus der EP 2 288 392 A1 (US 2011/282128 A1) bekannt. Als rotierendes Förderelement kann beispielsweise, wie in der EP 2 288 392 A1 beschrieben, ein Rotor mit ausklappbaren Propellern Verwendung finden, der am distalen Ende des Katheters vorgesehen ist. Ebenso ist denkbar, dass anders ausgebildete Förderelemente, wie beispielsweise helixartig ausgebildete Wendel Verwendung finden können.

Katheterpumpen werden als temporäres Kreislaufunterstützungssystem in das arterielle Gefäßsystem wie zum Beispiel die Aorta von Patienten eingesetzt, insbesondere dann, wenn das natürliche Herz nicht in der Lage ist, den Körper mit ausreichend Sauerstoff versetztem Blut zu versorgen. Das Förderelement und die Rotorwelle werden dabei mit vergleichsweise hohen Drehzahlen im Bereich von 7000 bis 15000 Umdrehungen betrieben. Der Pumpenkopf der Katheterpumpe kann mehrere Tage im Patienten verbleiben.

Der vorliegenden Erfindung liegt die Aufgabe zugrunde, eine eingangs beschriebene Katheterpumpe bereitzustellen, mit der ein funktionssicheres Expandieren des Käfigs erfolgen kann.

Diese Aufgabe wird mit einer Katheterpumpe mit den Merkmalen des Anspruchs 1 gelöst. Erfindungsgemäß ist folglich vorgesehen, dass der Betätigungsabschnitt ein in axialer Richtung mit dem Innenkatheter bewegungsgekoppeltes Grundteil und ein relativ zum Grundteil in axialer Richtung bewegbares, im oder am Grundteil geführt angeordnetes Betätigungsteil aufweist, wobei das proximale Ende des Außenkatheters mit dem Betätigungsteil und das distale Ende des Außenkatheters mit der proximalen Hülse in axialer Richtung derart bewegungsgekoppelt sind, dass beim Bewegen des Betätigungsteils weg vom Grundteil die proximale Hülse hin zur distalen Hülse bewegt wird.

Dadurch, dass das Betätigungsteil in oder am Grundteil geführt angeordnet ist, kann eine sichere Relativbewegung zwischen Grundteil und Betätigungsteil gewährleistet werden. Zum anderen kann dadurch, dass das proximale Ende des Außenkatheters mit dem Betätigungsteil und das distale Ende des Außenkatheters mit der proximalen Hülse in axialer Richtung bewegungsgekoppelt sind, durch Betätigen des Betätigungsteils relativ zum Grundteil die proximale Hülse in distaler Richtung auf die distale Hülse zubewegt werden, so dass die Filamente des Käfigs nach radial außen expandieren, um einen Förderelement umgebenden Raum bereitzustellen. Das Grundteil ist dabei vorzugsweise proximal zum Betätigungsteil angeordnet, d.h. das Betätigungsteil befindet sich zwischen dem Grundteil und dem Pumpenkopf. Vorzugsweise wird beim Verschieben des Betätigungsteils hin zum Grundteil nicht nur der Käfig expandiert, sondern zeitgleich oder zeitlich kurz danach, das Förderelement aus seiner kollabierten bzw. eingeklappten Lage in die expandierte Lage betätigt.

Zur ausreichenden Schmierung und Spülung der Lagerstellen des Förderelements ist vorgesehen, dass das Betätigungsteil einen Einlauf für Spülflüssigkeit aufweist und wenn das Grundteil einen Auslauf für die von den Lagerstellen kommende Spülflüssigkeit aufweist. Insofern kann über das Betätigungsteil, bzw. über dessen Einlauf, Spülflüssigkeit in den Katheter gepumpt und zu den Lagerstellen geleitet werden. Über das Grundteil, bzw. dessen Auslauf, kann zumindest ein Teil der eingebrachten Spülflüssigkeit aus dem Katheter abgeführt werden.

Dabei ist vorteilhaft, wenn das Betätigungsteil einen mit dem Einlauf verbundenen Einlaufraum aufweist, wobei der Innenkatheter sich durch den Einlaufraum erstreckt und der Einlaufraum mit einem zwischen dem Außenkatheter und dem Innenkatheter vorhandenen Einlauflumen verbunden ist, so dass durch den Einlauf kommende Spülflüssigkeit über den Einlaufraum und das Einlauflumen hin zu den Lagerstellen des Förderelements strömen kann. Dadurch kann erreicht werden, dass die Spülflüssigkeit nicht mit der im Innenkatheter rotierenden Welle in Kontakt kommt. Eine Verunreinigung der Spülflüssigkeit kann damit unterbunden werden. Der Innenkatheter mit darin im Betrieb rotierender Rotorwelle kann dennoch sicher durch den Einlaufraum hindurch zum Grundteil geführt werden.

Um dennoch eine Relativbewegung zwischen Betätigungsteil und Grundteil zu ermöglichen, ist vorteilhaft, wenn der Einlaufraum auf der proximalen Seite von einem im Einlaufraum axial verschiebbaren Kolbenabschnitt des Grundteils begrenzt ist. Dieser Kolbenabschnitt nimmt vorteilhafterweise das proximale Ende des Innenkatheters auf, durch den sich die Rotorwelle hin zum proximalen Ende des Grundteils erstreckt. Durch Vorsehen des Kolbenabschnitts kann erreicht werden, dass keine Spülflüssigkeit in proximaler Richtung aus dem Betätigungsteil strömen kann; dennoch kann das Betätigungsteil relativ zum Grundteil in axialer Richtung bewegt werden. Vorzugsweise weist der Kolbenabschnitt auf seiner radialen Außenseite einen umlaufenden Dichtring auf, der zum einen ein axiales Verschieben ermöglicht und zum anderen die Dichtheit des Einlaufraums sicherstellt. Der Einlaufraum ist dabei vorteilhafterweise ein möglichst kleines Volumen auf, um das Volumen des Einlaufraums bei einer Betätigung des Betätigungsabschnitts möglichst nur wenig zu verändern.

Weiterhin ist vorteilhaft, wenn das Grundteil einen hülsenartig ausgebildeten Lagerabschnitt mit einem Aufnahmeraum aufweist, in dem Lagerstellen zur Drehlagerung des proximalen Endes der Rotorwelle vorgesehen sind. Das Grundteil sieht folglich an seinem distalen Abschnitt den Kolbenabschnitt und in proximaler Richtung daran anschließend den Lagerabschnitt vor. Die Rotorwelle erstreckt sich dabei in axialer Richtung durch den Aufnahmeraum. Die Rotorwelle als solches kann dabei einteilig oder mehrteilig ausgebildet sein. Um eine gute Lagerung des proximalen Endes der Rotorwelle zu erreichen, ist vorteilhaft, wenn diese im Aufnahmeraum starr ausgebildet ist. Im Katheter hingegen kann die Rotorwelle flexibel ausgebildet sein, so dass der Katheter samt Rotorwelle eine gewisse Flexibilität aufweist. Im Aufnahmeraum kann eine Lagerhülse vorgesehen sein, in der Lagerstellen in Form von Drehlagern, insbesondere in Form von Gleit- Wälzlagerringen, fixiert sind. Vorzugsweise sind zwei axial zueinander beabstandete Lagerstellen vorgesehen, um eine sichere Lagerung des proximalen Endes der Rotorwelle in insbesondere axialer und radialer Richtung zu gewährleisten. Zum Abführen der Spülflüssigkeit und zum Schmieren der in dem Innenkatheter rotierenden Rotorwelle ist vorteilhaft, wenn der Aufnahmeraum mit einem zwischen dem Innenkatheter und der Rotorwelle vorhandenen Auslauflumen verbunden ist, so dass durch das Auslauflumen kommende Spülflüssigkeit über den Aufnahmeraum hin zum Auslauf strömen kann.

Die Anordnung ist dabei derart, dass die Spülflüssigkeit, die aus dem Auslauflumen in den Aufnahmeraum einströmt, die Lagerstellen, mit denen das proximale Ende der Rotorwelle gelagert ist, zur Kühlung, Spülung und Schmierung durchströmt. Die Drehlager können dabei Ausnehmungen, beispielsweise in Form von sich in axialer Richtung erstreckenden Bohrungen aufweisen, durch die die Spülflüssigkeit geleitet werden kann

Das Grundteil sieht ferner vorteilhafterweise an seinem proximalen Ende einen mit dem Auslauf verbundenen Auslaufraum vor, wobei die vom Aufnahmeraum kommende Spülflüssigkeit, nachdem sie vorteilhafterweise die Lagerstellen durchströmt hat, durch den Auslaufraum in den Auslauf abfließt.

Das proximale, freie Ende der Rotorwelle sieht dabei vorzugsweise einen im Auslaufraum vorgesehenen Drehkoppelabschnitt vor. Der Drehkoppelabschnitt ist dabei über Magnetelemente mit einem Antrieb berührungslos drehkoppelbar.

Zur weiteren, sicheren gegenseitigen Führung von Betätigungsteil und Grundteil ist vorteilhaft, wenn das Betätigungsteil an seinem proximalen Ende eine zylinderförmige Schiebeaufnahme für einen kolbenartigen Schiebeabschnitt des Grundteils aufweist. Dadurch kann ein sicheres axiales Betätigen von Betätigungsteil und Grundteil erreicht werden, ohne dass ein Abknicken der Teile beim Aufeinanderzubewegen erfolgen kann.

Der Schiebeabschnitt wird dabei vorteilhafterweise abschnittsweise von der Außenseite des Lagerabschnitts gebildet.

Weitere Vorteile und vorteilhafte Ausgestaltungen der Erfindung sind der nachfolgenden Beschreibung zu entnehmen, anhand derer ein Ausführungsbeispiel für die Erfindung näher beschrieben und erläutert ist.

Es zeigen:
Figur 1 eine Katheterpumpe mit einer Antriebseinheit und einem Katheter;
Figur 2 einen Längsschnitt durch den Betätigungsabschnitt der Katheterpumpe gemäß Figur 1 in nicht betätigter Einführlage des Pumpenkopfes; und
Figur 3 den Betätigungsabschnitt gemäß Figur 1 in betätigter Betriebslage.

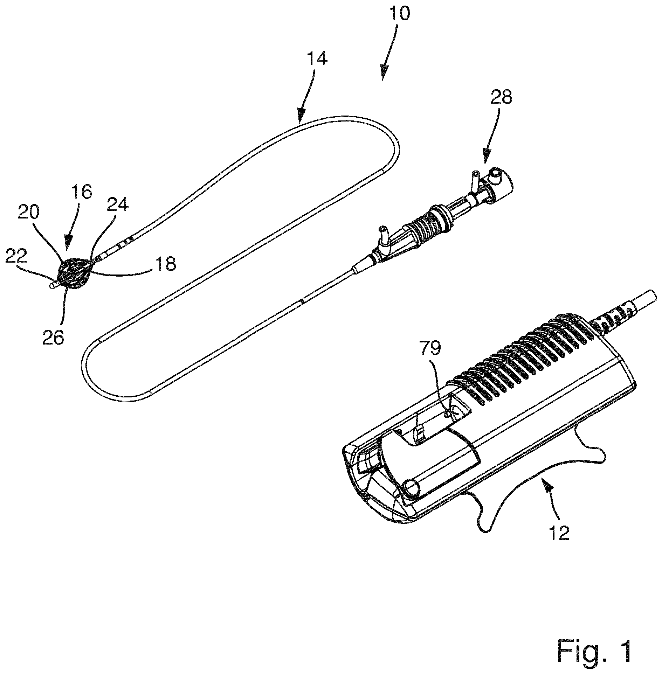

In der Figur 1 ist eine Katheterpumpe 10 sowie eine Antriebseinheit 12 zum Antreiben der Katheterpumpe 10 gezeigt. Die Katheterpumpe 10 weist einen Katheter 14 und am distalen Ende einen Pumpenkopf 16 zum Einführen in insbesondere die Aorta oder das Herz auf. Im Katheter 14 ist eine Rotorwelle vorgesehen, mittels der ein im Pumpenkopf 16 vorgesehenes Förderelement 18, beispielsweise einen Rotor mit ausklappbaren Propellern, in Rotation versetzbar ist. Das Förderelement 18 ist dabei von einem Käfig 20 umgeben, der eine distale Hülse 22 und eine proximale Hülse 24 sowie zwischen den Hülsen verlaufende Filamente 26 aufweist. Zum Expandieren des Käfigs 20 und auch des Förderelements 18 wird die proximale Hülse 24 in axialer Richtung hin zur distalen Hülse 22 bewegt.

An seinem proximalen Ende sieht der Katheter 14 einen Betätigungsabschnitt 28 vor, über den die proximaleHülse 24 hin zur distalen Hülse 22 bewegt werden kann. Ferner kann der Betätigungsabschnitt 28 in die Antriebseinheit 12 eingelegt werden, mittels welcher letztlich die Rotorwelle, und damit das Förderelement 18, in Rotation versetzbar ist.

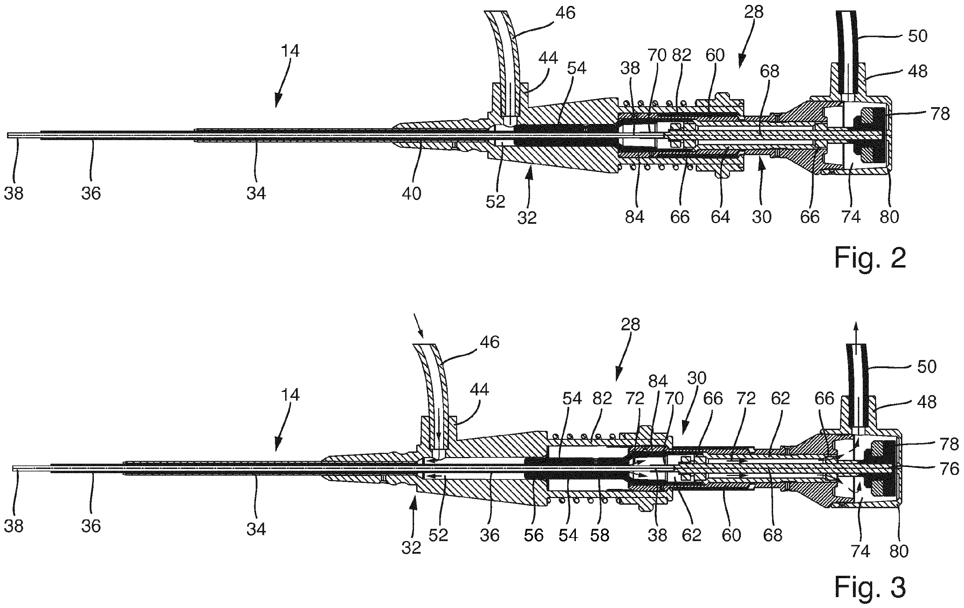

In den Schnitten gemäß Figur 2 und 3 ist der Betätigungsabschnitt 28 gezeigt, der ein Grundteil 30 sowie ein relativ zum Grundteil 30 in axialer Richtung bewegbares Betätigungsteil 32 umfasst. Wie ferner deutlich wird, weist der Katheter 14 einen Außenkatheter 34 und einen im Außenkatheter 34 axial verschiebbaren Innenkatheter 36 auf.

Im Innenkatheter 36 ist die rotierbare Rotorwelle 38 vorgesehen.

Zum Ausklappen des Käfigs 20 bzw. des Förderelements 18 wird folglich ausgehend von dem in Figur 2 gezeigten Grundzustand das Betätigungsteil 32 in distaler Richtung weg vom Grundteil 30 bewegt, bis es schließlich den in Figur 3 gezeigten Betätigungszustand einnimmt, in dem der Käfig 20, bzw. dessen Filamente 26, ausgeklappt sind. In diesem Betätigungszustand befindet sich der Betätigungsabschnitt 28 in der in Figur 1 gezeigten Antriebseinheit 12. Über einen dort vorhandenen Antriebsmotor kann dann die Rotorwelle, wie weiter unten beschrieben, zum Antreiben des Förderelements 18 angetrieben werden.

Der Außenkatheter 34 ist mit seinem proximalen Ende 40 fest am Betätigungsteil 32 angeordnet. Der Innenkatheter 36 durchgreift in axialer Richtung das Betätigungsteil 32 und ist mit seinem proximalen Ende 58 fest am Grundteil 30 angeordnet.

Durch axiales Betätigen des Betätigungsteils 32 relativ zum Grundteil 30 kann folglich der Außenkatheter 34 relativ zum Innenkatheter 36 bewegt werden. Dadurch, dass die distale Hülse 22 des Käfigs 20 mit dem Innenkatheter 36 bewegungsgekoppelt ist und die proximale Hülse 24 mit dem Außenkatheter in axialer Richtung bewegungsgekoppelt ist, kann durch eine Relativbewegung des Betätigungsteils 32 bezüglich des Grundteils 30, und damit des Außenkatheters 34 bezüglich des Innenkatheters 36, die proximale Hülse 24 hin zur distalen Hülse 22 bzw. weg von der distalen Hülse 22 zum Expandieren des Käfigs 20 bzw. zum Kollabieren oder Einklappen des Käfigs 20 bewegt werden. Die Bewegungskopplung ist dabei derart, dass nicht nur der Käfig 20, sondern zeitgleich mit dem Käfig 20, oder vorzugsweise kurz danach, das Förderelement 18 beim Bewegen des Betätigungsteils 32 in distaler Richtung expandiert wird.

Wie aus Figur 1 und 2 deutlich wird, weist das Betätigungsteil 32 einen Einlauf 44 auf, in welchem sich ein Schlauch 46 befindet, über den Spülflüssigkeit an die Lagerstellen des Förderelements 18 gepumpt werden kann. Entsprechend befindet sich am Grundteil 30 ein Auslauf 48, in welchem sich ein Schlauch 50 befindet, über den von den Lagerstellen des Förderelements 18 kommende Spülflüssigkeit abgeführt werden kann.

Im Betrieb strömt folglich Spülflüssigkeit, wie in Figur 3 gezeigt, über den Schlauch 46 in den Einlauf 44. Im Betätigungsteil 32 ist ein mit dem Einlauf 44 verbundener Einlaufraum 52 vorgesehen, der mit einem zwischen dem Außenkatheter 34 und dem Innenkatheter 36 vorhandenen Einlauflumen verbunden ist. Dadurch kann Spülflüssigkeit vom Einlaufraum 52 über das Einlauflumen hin zu den Lagerstellen des Förderelements 18 am distalen Ende des Katheters 14 strömen.

Wie aus den Figuren 2 und 3 deutlich wird, ist der Einlaufraum 52 auf der proximalen Seite von einem in dem Einlaufraum 52 axial verschiebbaren und in den Einlaufraum 52 eintauchenden Kolbenabschnitt 54 des Grundteils 30 begrenzt. Um eine ausreichende Dichtung zwischen dem freien Ende des Kolbenabschnitts 54 und der Wandung des Einlaufraums 52 zu ermöglichen, ist am Kolbenabschnitt 54 in einer umlaufenden Nut ein Dichtring 56 vorgesehen.

Der Kolbenabschnitt 54 nimmt zudem das proximale Ende 58 des Innenkatheters 36 auf. Dadurch ist das proximale Ende 58 des Innenkatheters 36 in axialer Richtung bewegungsgekoppelt mit dem Kolbenabschnitt 54 verbunden.

Das Grundteil 30 weist zudem einen hülsenartig ausgebildeten Lagerabschnitt 60 auf, der einen Aufnahmeraum 62 bildet. Im Aufnahmeraum 62 ist eine Lagerhülse 64 untergebracht, in der zwei axial zueinander beabstandet Drehlager 66 vorgesehen sind, welche den proximalen Endabschnitt 68 der Rotorwelle 38 drehlagern. Die Rotorwelle 38 bzw. deren Endabschnitt 68 erstreckt sich dabei durch den Aufnahmeraum 62. Der Endabschnitt 68 der Rotorwelle 38 ist dabei als starre Welle ausgebildet, die über einen Kopplungsabschnitt 70 mit dem flexiblen Teil der Rotorwelle 38 verbunden ist.

Der Aufnahmeraum 62 ist zudem mit einem zwischen dem Innenkatheter 36 und der Rotorwelle 38 vorhandene Auslauflumen verbunden. An seinem proximalen Ende weist das Grundteil 30 einen mit dem Auslauf 48 verbundenen Auslaufraum 74 auf, der mit dem Auslauf 48 bzw. den Schlauch 50 verbunden ist. Damit kann die von den Lagerstellen des Förderelements 18 kommende Spülflüssigkeit über das Auslauflumen, den Aufnahmeraum 62 und den Auslaufraum 74 hin zum Auslauf 48 bzw. Schlauch 50, wie mit den Pfeilen Zeile 72 angedeutet, strömen. Hierdurch wird zum einen die sich im Betrieb drehende Rotorwelle 38 im Innenkatheter 36 geschmiert und zum anderen können die Drehlager 66 ausreichend geschmiert und gekühlt werden.

Am freien, proximalen Ende 76 der Rotorwelle 38 bzw. an deren Endabschnitt 68 ist ein Drehkoppelabschnitt 78 vorgesehen. Der Drehkoppelabschnitt 78 umfasst dabei einen Magnetring, der über einen antriebsseitigen, in Figur 1 angedeuteten Magnetring 79 über magnetische Kopplung in Rotation versetzt werden kann. An der Rotorwelle 38 bzw. an deren Endabschnitt 68 kann ein weiterer Magnet vorgesehen sein, mit dem über einen entsprechenden Sensor in der Antriebseinheit detektiert werden kann, ob die Welle sich dreht oder nicht, bzw. mit welcher Drehzahl sie sich dreht.

Wie ebenfalls aus Figur 2 und 3 deutlich wird, weist das Grundteil 30 an seinem proximalen Ende eine Kappe 80 auf, die den Auslaufraum 74 abdeckt und welche zudem den Auslauf 48 beinhaltet.

Zur sicheren Führung des Betätigungsteils 32 gegenüber dem Grundteil 30 ist am proximalen Ende des Betätigungsteils 32 eine zylinderförmige Schiebeaufnahme 82 vorgesehen. In der Schiebeaufnahme 82 ist ein kolbenartig ausgebildeter Schiebeabschnitt 84 des Grundteils 30 verschiebbar untergebracht. Der Schiebeabschnitt 84 wird dabei von Abschnitten der Mantelfläche des Lagerabschnitts 60 gebildet. Durch Vorsehen des Schiebeabschnitts 84 und der zugehörigen Schiebeaufnahme 82 kann ein sicheres axiales Bewegen des Betätigungsteils 32 auf dem Grundteil 30 ermöglicht werden.

Das Grundteil 30 sowie das Betätigungsteil 32 können dabei einstückig oder mehrstückig ausgebildet sein. Bei dem in der Figur gezeigten Ausführungsbeispiel besteht das Grundteil 30 aus mehreren Einzelteilen, wie beispielsweise dem Kolbenabschnitt 54, dem Lagerabschnitt 60 samt Lagerhülse 64 und der Kappe 80.

## Patentansprüche

1. Katheterpumpe (10), mit einem Katheter (14),
mit einem am distalen Ende des Katheters vorgesehenen Pumpenkopf (16) zum Einführen in das arterielle Gefäßsystem,
wobei der Katheter (14) einen Außenkatheter (34) und einen im Außenkatheter (34) angeordneten Innenkatheter (36) aufweist, mit einer im Innenkatheter (36) verdrehbar angeordneten Rotorwelle (38) zum Antreiben eines am Pumpenkopf (16) vorgesehenen, expandierbaren Förderelements (18),
mit einem am proximalen Ende des Katheters (14) vorgesehenen Betätigungsabschnitt (28), über den die Rotorwelle (38) antreibbar ist, mit einem das Förderelement (18) umgebenden Käfig (20), wobei der Käfig (20) eine distale und eine proximale Hülse (22, 24) sowie zwischen den Hülsen (22, 24) verlaufende Filamente (26) aufweist, wobei die proximale Hülse (24) zum Expandieren des Käfigs (20) in axialer Richtung hin zur distalen Hülse (22) bewegt werden kann, **dadurch gekennzeichnet,**
**dass** der Betätigungsabschnitt (28) ein in axialer Richtung mit dem Innenkatheter (36) bewegungsgekoppeltes Grundteil (30) und ein relativ zum Grundteil (30) in axialer Richtung bewegbares, im oder am Grundteil (30) geführt angeordnetes Betätigungsteil (32) aufweist, wobei das proximale Ende (40) des Außenkatheters (34) mit dem Betätigungsteil (32) und das distale Ende des Außenkatheters mit der proximalen Hülse (24) in axialer Richtung derart bewegungsgekoppelt sind, dass beim Bewegen des Betätigungsteils (32) weg vom Grundteil (30) die proximale Hülse (24) hin zur distalen Hülse (22) bewegt wird, und
**dass** das Betätigungsteil (32) einen Einlauf (44) für Spülflüssigkeit zur Weiterleitung an Lagerstellen des Förderelements (18) aufweist und dass das Grundteil (30) einen Auslauf (48) für von den Lagerstellen kommende Spülflüssigkeit aufweist.

2. Katheterpumpe (10) nach Anspruch 1, **dadurch gekennzeichnet, dass** das Betätigungsteil (32) einen mit dem Einlauf (44) verbundenen Einlaufraum (52) aufweist, wobei der Innenkatheter (36) sich durch den Einlaufraum (52) erstreckt und der Einlaufraum (52) mit einem zwischen dem Außenkatheter (34) und dem Innenkatheter (36) vorhandenen Einlauflumen verbunden ist, so dass durch den Einlauf (44) kommende Spülflüssigkeit über den Einlaufraum (52) und das Einlauflumen hin zu den Lagerstellen des Förderelements (18) strömen kann.

3. Katheterpumpe (10) nach Anspruch 2, **dadurch gekennzeichnet, dass** der Einlaufraum (52) auf der proximalen Seite von einem im Einlaufraum (52) axial verschiebbaren Kolbenabschnitt (54) des Grundteils (30) begrenzt ist, an dem das proximale Ende (58) des Innenkatheters (36) befestigt ist und durch den sich die Rotorwelle (38) erstreckt.

4. Katheterpumpe (10) nach wenigstens Anspruch 1, **dadurch gekennzeichnet, dass** das Grundteil (30) einen hülsenartig ausgebildeten Lagerabschnitt (60) mit einem Aufnahmeraum (62) aufweist, in dem Lagerstellen (66) zur Drehlagerung des proximalen Endes der Rotorwelle (38, 68) vorgesehen sind.

5. Katheterpumpe (10) nach Anspruch 4, **dadurch gekennzeichnet, dass** im Aufnahmeraum (62) eine Lagerhülse (64) vorgesehen ist, in der die Lagerstellen in Form von Drehlagern (66) fixiert sind.

6. Katheterpumpe (10) nach Anspruch 5, **dadurch gekennzeichnet, dass** die Drehlager Ausnehmungen aufweisen, durch die die Spülflüssigkeit geleitet werden kann.

7. Katheterpumpe (10) nach Anspruch 5 oder 6, **dadurch gekennzeichnet, dass** der Aufnahmeraum (62) mit einem zwischen dem Innenkatheter (36) und der Rotorwelle (38) vorhandenen Auslauflumen verbunden ist, so dass durch das Auslauflumen kommende Spülflüssigkeit über den Aufnahmeraum (62) hin zum Auslauf (48) strömen kann.

8. Katheterpumpe (10) nach wenigstens Anspruch 1, **dadurch gekennzeichnet, dass** das Grundteil (30) an seinem proximalen Ende einen mit dem Auslauf (48) verbundenen Auslaufraum (74) aufweist, wobei die vom Aufnahmeraum (62) kommende Spülflüssigkeit durch den Auslaufraum (74) in den Auslauf (48) abfließt.

9. Katheterpumpe (10) nach einem der vorhergehenden Ansprüche, **dadurch gekennzeichnet, dass** das proximale, freie Ende der Rotorwelle (38) einen Drehkoppelabschnitt (78) aufweist.

10. Katheterpumpe (10) nach einem der vorhergehenden Ansprüche, **dadurch gekennzeichnet, dass** das Betätigungsteil an seinem proximalen Ende eine zylinderförmige Schiebeaufnahme (82) für einen kolbenartigen Schiebeabschnitt (84) des Grundteils (30) aufweist.

11. Katheterpumpe (10) nach Anspruch 10, **dadurch gekennzeichnet, dass** der Schiebeabschnitt (84) wenigstens abschnittsweise vom Lagerabschnitt (60) gebildet wird.

## Claims

1. Catheter pump (10) comprising a catheter (14),
a pump head (16) provided at the distal end of the catheter for insertion into the arterial vascular system,
the catheter (14) having an outer catheter (34) and an inner catheter (36) arranged in the outer catheter (34), comprising a rotor shaft (38) which is rotatably arranged in the inner catheter (36) for driving an expandable conveying element (18) provided on the pump head (16),
an actuation portion (28) which is provided at the proximal end of the catheter (14) and by means of which the rotor shaft (38) can be driven, and comprising a cage (20) surrounding the conveying element (18), the cage (20) having a distal and a proximal sleeve (22, 24) and filaments (26) extending between the sleeves (22, 24), the proximal sleeve (24) being movable in the axial direction toward the distal sleeve (22) in order to expand the cage (20), **characterized in that**
the actuation portion (28) has a base part (30) which is movement-coupled to the inner catheter (36) in the axial direction and an actuation part (32) which is movable in the axial direction relative to the base part (30) and is guided in or on the base part (30), the proximal end (40) of the outer catheter (34) being movement-coupled to the actuation part (32) and the distal end of the outer catheter being movement-coupled to the proximal sleeve (24) in the axial direction such that the proximal sleeve (24) is moved toward the distal sleeve (22) when the actuation part (32) moves away from the base part (30), and
**in that** the actuation part (32) has an inlet (44) for rinsing liquid to be conveyed to bearing points of the conveying element (18) and **in that** the base part (30) has an outlet (48) for rinsing liquid coming from the bearing points.

2. Catheter pump (10) according to claim 1, **characterized in that** the actuation part (32) has an inlet chamber (52) connected to the inlet (44), the inner catheter (36) extending through the inlet chamber (52) and the inlet chamber (52) being connected to an inlet lumen provided between the outer catheter (34) and the inner catheter (36), such that rinsing liquid coming through the inlet (44) can flow via the inlet chamber (52) and the inlet lumen to the bearing points of the conveying element (18).

3. Catheter pump (10) according to claim 2, **characterized in that** the inlet chamber (52) is delimited on the proximal side by a piston portion (54) of the base part (30) that is axially movable in the inlet chamber (52), to which portion the proximal end (58) of the inner catheter (36) is fastened and through which the rotor shaft (38) extends.

4. Catheter pump (10) according to at least claim 1, **characterized in that** the base part (30) has a sleeve-like bearing portion (60) having a receiving chamber (62) in which bearing points (66) are provided for rotationally mounting the proximal end of the rotor shaft (38, 68).

5. Catheter pump (10) according to claim 4, **characterized in that** a bearing sleeve (64) is provided in the receiving chamber (62), in which bearing sleeve the bearing points are fixed in the form of rotary bearings (66).

6. Catheter pump (10) according to claim 5, **characterized in that** the rotary bearings have recesses through which the rinsing liquid can be guided.

7. Catheter pump (10) according to claim 5 or claim 6, **characterized in that** the receiving chamber (62) is connected to an outlet lumen provided between the inner catheter (36) and the rotor shaft (38), such that rinsing fluid coming through the outlet lumen can flow via the receiving chamber (62) to the outlet (48).

8. Catheter pump (10) according to at least claim 1, **characterized in that** the base part (30) has, at the proximal end thereof, an outlet chamber (74) which is connected to the outlet (48), the rinsing liquid coming from the receiving chamber (62) flowing out via the outlet chamber (74) into the outlet (48).

9. Catheter pump (10) according to any of the preceding claims, **characterized in that** the proximal, free end of the rotor shaft (38) has a rotary coupling portion (78).

10. Catheter pump (10) according to any of the preceding claims, **characterized in that** the actuation part has, at the proximal end thereof, a cylindrical sliding receptacle (82) for a piston-like sliding portion (84) of the base part (30).

11. Catheter pump (10) according to claim 10, **characterized in that** the sliding portion (84) is formed at least in portions by the bearing portion (60).

## Revendications

1. Pompe à cathéter (10), avec un cathéter (14), avec une tête de pompe (16) prévue à l'extrémité distale du cathéter, à introduire dans le système vasculaire artériel,
dans laquelle le cathéter (14) présente un cathéter extérieur (34) et un cathéter intérieur (36) disposé dans le cathéter extérieur (34), avec un arbre de rotor (38) disposé en rotation dans le cathéter intérieur (36) pour l'entraînement d'un élément de refoulement (18) pouvant être en expansion, prévu sur la tête de pompe (16),
avec une section d'actionnement (28) prévue à l'extrémité proximale du cathéter (14), par l'intermédiaire de laquelle l'arbre de rotor (38) peut être entraîné, avec une cage (20) entourant l'élément de refoulement (18), dans laquelle la cage (20) présente un manchon distal et un manchon proximal (22, 24) ainsi que des filaments (26) s'étendant entre les manchons (22, 24), dans laquelle le manchon proximal (24) pour l'expansion de la cage (20) peut être déplacé dans la direction axiale vers le manchon distal (22), **caractérisé en ce**
**que** la section d'actionnement (28) présente une partie de base (30) accouplée en mouvement au cathéter intérieur (36) dans la direction axiale et une partie d'actionnement (32) mobile dans la direction axiale par rapport à la partie de base (30), disposée de manière guidée dans ou sur la partie de base (30), dans laquelle l'extrémité proximale (40) du cathéter extérieur (34) est accouplée en mouvement à la partie d'actionnement (32) et l'extrémité distale du cathéter extérieur est accouplée en mouvement au manchon proximal (24) dans la direction axiale, de telle sorte que lors du déplacement de la partie d'actionnement (32) à distance de la partie de base (30), le manchon proximal (24) est déplacé en direction du manchon distal (22), et que la partie d'actionnement (32) présente une entrée (44) pour un liquide de lavage à acheminer sur des points de palier de l'élément de refoulement (18) et que la partie de base (30) présente une sortie (48) pour un liquide de lavage provenant des points de palier.

2. Pompe à cathéter (10) selon la revendication 1, **caractérisée en ce que** la partie d'actionnement (32) présente une chambre d'entrée (52) reliée à l'entrée (44), dans laquelle le cathéter intérieur (36) s'étend à travers la chambre d'entrée (52) et la chambre d'entrée (52) est reliée à une lumière d'entrée présente entre le cathéter extérieur (34) et le cathéter intérieur (36), de sorte que le liquide de lavage venant par l'entrée (44) peut s'écouler en passant par la chambre d'entrée (52) et la lumière d'entrée en direction des points de palier de l'élément de refoulement (18).

3. Pompe à cathéter (10) selon la revendication 2, **caractérisée en ce que** la chambre d'entrée (52) est délimitée sur la face proximale par une section de piston (54) de la partie de base (30) pouvant coulisser axialement dans la chambre d'entrée (52), sur laquelle l'extrémité proximale (58) du cathéter intérieur (36) est fixée et à travers laquelle l'arbre de rotor (38) s'étend.

4. Pompe à cathéter (10) selon au moins la revendication 1, **caractérisée en ce que** la partie de base (30) présente une section de palier (60) réalisée à la façon d'un manchon avec une chambre de réception (62), dans laquelle des points de palier (66) pour le montage en rotation de l'extrémité proximale de l'arbre de rotor (36, 38) sont prévus.

5. Pompe à cathéter (10) selon la revendication 4, **caractérisée en ce qu'**un manchon de palier (64), dans lequel les points de palier sous forme de paliers rotatifs (66) sont fixés, est prévu dans la chambre de réception (62).

6. Pompe à cathéter (10) selon la revendication 5, **caractérisée en ce que** les paliers rotatifs présentent des évidements, par lesquels le liquide de lavage peut être acheminé.

7. Pompe à cathéter (10) selon la revendication 5 ou 6, **caractérisée en ce que** la chambre de réception (62) est reliée à une lumière de sortie présente entre le cathéter intérieur (36) et l'arbre de rotor (38), de sorte que le liquide de lavage venant par la lumière de sortie peut s'écouler en direction de la sortie (48) en passant par la chambre de réception (62).

8. Pompe à cathéter (10) selon au moins la revendication 1, **caractérisée en ce que** la partie de base (30) présente à son extrémité proximale une chambre de sortie (74) reliée à la sortie (48), dans laquelle le liquide de lavage venant de la chambre de sortie (62) s'évacue dans la sortie (48) par la chambre de sortie (74).

9. Pompe à cathéter (10) selon l'une quelconque des revendications précédentes, **caractérisée en ce que** l'extrémité libre proximale de l'arbre de rotor (38) présente une section d'accouplement en rotation (78).

10. Pompe à cathéter (10) selon l'une quelconque des revendications précédentes, **caractérisée en ce que** la partie d'actionnement présente à son extrémité proximale un logement coulissant cylindrique (82) pour une section coulissante (84) du type piston de la partie de base (30).

11. Pompe à cathéter (10) selon la revendication 10, **caractérisée en ce que** la section coulissante (84) est formée au moins sur certaines parties par la section de palier (60).
